(19)

Europäisches Patentamt
European Patent Office
Office européen des brevets

(11) **EP 3 368 681 B1**

(12) **EUROPEAN PATENT SPECIFICATION**

(45) Date of publication and mention
of the grant of the patent:
**22.05.2019 Bulletin 2019/21**

(21) Application number: **16797886.5**

(22) Date of filing: **17.11.2016**

(51) Int Cl.:
***C12Q 1/18*** (2006.01)

(86) International application number:
**PCT/EP2016/077918**

(87) International publication number:
**WO 2017/085153 (26.05.2017 Gazette 2017/21)**

(54) **ASSAY FOR DETERMINING ANTIBIOTICS IN WASTE**

ASSAY ZUR BESTIMMUNG VON ANTIBIOTIKA IN ABFÄLLEN

DOSAGE PERMETTANT DE DÉTECTER DES ANTIBIOTIQUES DANS LES DÉCHETS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.11.2015 EP 15195669
21.01.2016 EP 16152287**

(43) Date of publication of application:
**05.09.2018 Bulletin 2018/36**

(73) Proprietor: **Centrient Pharmaceuticals
Netherlands B.V.
2613 AX Delft (NL)**

(72) Inventors:
• **HANEMAAIJER, Leendert Marinus
6100 AA Echt (NL)**
• **JAGESAR, Dhiredj Chandre
6100 AA Echt (NL)**

(74) Representative: **de Pauw, Elmar Sebastian David
DSM Sinochem Pharmaceuticals Netherlands
B.V.
Att: IP Department
PP 9940850
Alexander Fleminglaan 1
2613 AX Delft (NL)**

(56) References cited:
**EP-A1- 2 226 389      WO-A1-2005/118837
CN-A- 101 633 948      GB-A- 1 467 439
US-A- 5 591 599**

• **MISAKO TAGIRI-ENDO ET AL: "Rapid
determination of five antibiotic residues in swine
wastewater by online solid-phase extraction-high
performance liquid chromatography-tandem
mass spectrometry", ANALYTICAL AND
BIOANALYTICAL CHEMISTRY, SPRINGER,
BERLIN, DE, vol. 393, no. 4, 9 December 2008
(2008-12-09), pages 1367-1375, XP019702567,
ISSN: 1618-2650**
• **ROSSMANN JULIA ET AL: "Simultaneous
determination of most prescribed antibiotics in
multiple urban wastewater by SPE-LC-M",
JOURNAL OF CHROMATOGRAPHY B:
BIOMEDICAL SCIENCES & APPLICATIONS,
ELSEVIER, AMSTERDAM, NL, vol. 969, 12 August
2014 (2014-08-12), pages 162-170, XP029057093,
ISSN: 1570-0232, DOI:
10.1016/J.JCHROMB.2014.08.008**

**Description**

**Field of the invention**

The present invention relates to a simple and easy-to-use method for the rapid determination of the presence of an antibiotic in a waste such as e.g. liquid or solid waste streams from plants. The present invention also relates to a kit comprising an assay and a manual for the rapid determination of the presence of an antibiotic in a waste.

**Background of the invention**

Antimicrobial resistance is one of the major global problems which no longer is a prediction for the future, but happening right now in every region of the world and has the potential to affect anyone, of any age, in any country. Antimicrobial resistance -when bacteria change so antibiotics no longer work in people who need them to treat infections- is now a major threat to public health. There have been increasing public calls for global collective action to address the threat, including a proposal for an international treaty on antimicrobial resistance. Antibiotic resistance is not properly mapped across the world, but the countries that are affected the most are poorer countries with weaker healthcare systems. There are three main ways by which antimicrobial resistance can occur: natural resistance to certain types of bacteria, genetic mutation, or by acquiring resistance from another bacterium.

Antimicrobial resistance can happen spontaneously due to mutations of the microbes themselves, to build up of resistance over time, or to misuse of antibiotics. Resistant microbes become increasingly difficult to treat, requiring alternative medications or higher doses, both of which may be more costly or more toxic to the individual. Some infections already are becoming untreatable due to antimicrobial resistance.

Antimicrobial resistance is an increasingly problematic issue that leads to millions of deaths every year. The rising trend in drug resistance can be attributed to four primary areas: use of antibiotics in the human population, use of antibiotics in the animal population, the spread of resistant strains between human and/or non-human sources, and release of antibiotics in the environment.

The latter cause for antimicrobial resistance, release of antibiotics in the environment, is a growing problem in various segments of agriculture and industry. In this respect, the spread and contamination of the environment, especially through "hot spots" such as hospital and industrial wastewater and untreated urban wastewater, presents a growing public health problem. Antibiotics have been polluting the environment since their introduction through several sources such as human waste (medication, farming), treatment of animals, and the pharmaceutical industry. Antibiotic waste may contain, or may be a source for development of, resistant bacteria. As a result antibiotic waste may result in introduction of antibiotic resistant bacteria into the environment. As bacteria replicate quickly, the resistant bacteria that enter the environment replicate their resistance genes as they continue to divide. In addition, bacteria carrying resistance genes (*i.e.* resistant bacteria) have the ability to spread those genes to other species via horizontal gene transfer. Therefore, even if the specific antibiotic is no longer introduced into the environment, antibiotic-resistance genes will persist through the bacteria that have since replicated without continuous exposure.

As one of the consequences, producers of antibiotics will have to ensure that the cleanest, most rigorous and sustainable methods are applied to minimize the environmental impact of producing life-saving medicines and a first step in this direction is to measure antibiotic content in (industrial) waste streams.

Technically, with state-of-the-art analytical methods this is possible. The amount of antibiotics in aqueous streams can be measured by means of several methods including photometry, electrochemistry, gas chromatography and direct liquid chromatography (Pettas and Karayannis (2004), Anal. Chim. Acta 522, 275-280). A drawback. A drawback is that these techniques are laborious and time-consuming and often need expensive devices and well-trained personnel. As a consequence these methods are difficult, if not impossible, to implement at high frequency and/or at many locations by personnel without particularly high technical skill.

Thus, a need exists for simple, inexpensive and easy-to-use methods for determining the presence and/or concentration of antibiotics in waste streams. The present disclosure provides such a method.

Microbiological assays for the determination of antibiotics have been known for quite some time. Examples of such assays are described in CA 2056581, DE 3613794, EP 0005891, EP 0285792, EP 2226389, GB 1467439, WO 94/18343 and WO 2005/118837. In majority these assays are designed for use in the dairy industry, notably for the analysis of milk. These microbiological assays include a ready-to-use assay that makes use of a microorganism and gives a result by a change indicated by an indicator, such as an indicator molecule that may give an indicator signal, added to the assay medium. The principle is that when antibiotic is present in the sample in a concentration sufficient to inhibit the growth of the microorganism the color of the indicator will stay the same, while, when no inhibition occurs, the growth of the microorganism is accompanied by the formation of acid and/or reduced metabolites or other phenomena that will induce a change in the indicator signal of the indicator.

Unfortunately, when such assays are applied on substrates that are more complex than milk, such as wastewater streams that comprise many other components or even (semi-)solid wastes such as filter cakes, mycelia or concentrated residues from fermentation processes, the results are difficult to interpret and consequently unreliable. If the indicator change is a change in color of the indicator molecule, such change is often difficult to observe or interpret. Another possible drawback is that additional, non-antimicrobial, components in complex samples influence the proper functioning of the assay microorganism.

## Summary of the invention

The present disclosure relates to a method for determining the presence or absence of an antibiotic in a lactose-free sample that is essentially lactose-free and/or contains less than 0.01% (w/w) of lactose comprising the steps of:

(a) contacting said lactose-free sample with an assay medium which comprises a microorganism, a gelling agent and an indicator capable of detecting growth or inhibition of the microorganism to obtain an assembly of the lactose-free sample and the assay medium;
(b) incubating the assembly obtained in step (a) for a period of time sufficient to grow the microorganism in case no antibiotic is present in said lactose-free sample; and
(c) detecting growth or inhibition of growth of the microorganism with the indicator, characterized in that milk and/or powdered milk is added in step (a).

The disclosure further relates to a kit comprising:

(a) a container with assay medium which comprises a microorganism, a gelling agent and an indicator capable of detecting growth or inhibition of the microorganism to obtain a mixture;
(b) a manual comprising instructions for determining the concentration of an antibiotic in a lactose-free sample that is essentially lactose-free and/or contains less than 0.01% (w/w) of lactose.
(c) a container comprising powdered milk.

The disclosure further relates to the use of Bromocresol Purple or Bromothymol Blue for determining the concentration of an antibiotic in a lactose-free sample that is essentially lactose-free and/or contains less than 0.01% (w/w) of lactose, wherein milk and/or powdered milk is added to said lactose-free sample. The disclosure further relates to the use of a β-lactam degrading compound for determining the concentration of an antibiotic in a lactose-free sample that is essentially lactose-free and/or contains less than 0.01% (w/w) of lactose, wherein milk and/or powdered milk is added to said lactose-free sample.

## Detailed description of the invention

It is an object of the present invention to provide a simple, inexpensive and easy-to-use method for determining antibiotics in waste. Also provided is a kit of parts comprising an easy-to-use microbiological assay and instructions for application on waste, including complex matrices such as wastewater streams that comprise many other components or even (semi-)solid wastes such as filter cakes, mycelia or concentrated residues from fermentation processes.

The present disclosure provides a unique reliable and simple versatile testing protocol.

In the context of the present invention, the terms and abbreviations are defined as follows.

The term "antibiotic" refers to compounds such as *e.g.* β-lactams, tetracyclines, aminoglycosides, quinolones and sulfonamides and derivatives thereof; and any combination thereof. Examples of antibiotics the presence of which may be detected with the method or kit of the present invention are, but are not limited to aminoglycosides (such as amikacin, dibekacin, gentamicin, kanamycin A, neomycins B, C and E, netilmicin, sisomicin, streptomycin, tobramycin and the like), cephalosporins (such as 7-aminocephalosporanic acid, 7-aminodesacetoxycephalosporanic acid, cefaclor, cefadroxil, cefamandole, cefatrizine, cefazolin, cefbuperazone, cefcapene, cefdinir, cefditoren, cefepime, cefetamet, cefixime, cefmenoxime, cefmetazole, cefminox, cefodizime, cefonicid, cefoperazone, ceforanide, cefoselis, cefotaxime, cefotiam, cefotetan, cefovecin, cefoxitin, cefozopran, cefpiramide, cefpirome, cefpodoxime, cefprozil, cefquinome, cefroxadine, cefsulodin, ceftaroline, ceftazidime, cefteram, ceftibuten, ceftiofur, ceftizoxime, ceftobiprole, ceftolozane, ceftriaxone, cefuroxime, cephalexin, cephalosporin C, cephradine and the like), penicillins (such as 6-aminopenicillanic acid, amoxicillin, ampicillin, cloxacillin, flucloxacillin, oxacillin, penicillin G, penicillin V and the like), first-generation quinolones (such as cinoxacin, nalidixic acid, oxolinic acid, pipemidic acid, piromidic acid, rosoxacin), second-generation quinolones (such as ciprofloxacin, enoxacin, fleroxacin, lomefloxacin, nadifloxacin, norfloxacin, ofloxacin, pefloxacin, rufloxacin) third-generation quinolones (such as balofloxacin, grepafloxacin, levofloxacin, pazufloxacin, sparfloxacin, temafloxacin, tosufloxacin), fourth-generation quinolones (such as clinafloxacin, gatifloxacin, gemifloxacin, moxifloxacin, sitafloxacin, trovafloxacin, prulifloxacin), sulfonamides and tetracyclines (such as chlortetracycline, demeclocycline, lymecycline, meclocycline, methacycline, minocycline, oxytetracycline, rolitetracycline, tetracycline and the like). Importantly, also included are degradation products and or re-arranged derivatives of the above compounds that still possess antimicrobial activity.

The term "assay medium" refers to a composition in the form of a solution, a solid or, preferably, in the form of a gel-like matrix like a sol or a gel. If the assay medium has the form of a gel-like matrix, it may comprise a gelling agent. The person skilled in the art will understand that a solid assay media may be based on carrier materials such as ceramics, cotton, glass, metal particles, paper, polymers in any shape or form, a silicates, sponges, wool and the like. Usually, an assay medium comprises one or more indicators. The assay medium may comprise one or more types of microorganisms or enzymes as detecting agents and at least one nutrient. The assay medium may have the form of a tablet, disc or paper filter comprising the microorganism, indicator and nutrient. These three constituents may be present in a single tablet, but also in two or more tablets. Of course, assays combining assay media in solid, liquid and/or gel-like form may also be used. In an embodiment a microorganism, an indicator and a nutrient are introduced into an agar solution. The agar solution is allowed to solidify to form the assay medium such that the microorganism stays alive, but cannot multiply because of $e.g.$ low temperature. In an embodiment the amount of gelling agent in the assay medium is between 2 and 100 $g.l^{-1}$, preferably between 5 and 50 $g.l^{-1}$, more preferably between 10 and 20 $g.l^{-1}$, most preferably between 12 and 15 $g.l^{-1}$. In a preferred embodiment the gelling agent is agar. Optionally, the assay medium may also contain one or more buffers, stabilizers, surfactant, salts, substances that change the sensitivity to certain antimicrobial compounds in a positive (improve sensitivity) or negative (decrease sensitivity) way, viscosity-increasing agents or any combination thereof. When a buffer is present in the medium, it may be added during the mixing of the components of the medium or the components may be dissolved and/or suspended in the buffer. Suitable buffering agents include, but are not limited to, alanine, potassium phosphate and sodium phosphate. Examples of substances that change the sensitivity to certain antimicrobial compounds are antifolates like ormethoprim, tetroxoprim and trimethoprim that improve the sensitivity of the microorganism towards sulfa compounds or salts of oxalic acid or hydrofluoric acid or calcium chelating agents which improve the sensitivity towards tetracycline. Cysteine and penicillin binding protein are compounds that are known to decrease the sensitivity to certain antimicrobial compounds. Examples of viscosity-increasing agents include, but are not limited to, ascorbyl methylsilanol pectinate, carbomer, carboxymethyl cellulose, cetearyl alcohol, cetyl alcohol, cetyl esters, cocamide DEA, emulsifying wax, glucose, hydroxyethyl cellulose, hydroxypropylmethyl cellulose, lauramide DEA, linoleamide DEA, magnesium aluminum silicate, maltodextrins, PEG-8 distearate, polyacrylamide, polyvinyl alcohol, PVP/hexadecene copolymer, sodium chloride, sodium sulfate, soyamidopropyl betaine, xanthan gum and the like. A suitable stabilizer is $e.g.$ colloidal silica. Alternatively, the optional ingredients of the assay medium mentioned above may also be added exogenously. The assay medium may be contained within any type of container; frequently used containers are tubes, microtiter plates and petri dishes. The containers may be of any shape and size and from any material available, provided that observation of indicator changes is possible. Observation of indicator changes may be performed visually, but can also be performed using a sample-reading device such as a scanner. Optionally, means for sealing of said containers filled with assay medium during incubation and/or an insert with instructions for use and/or a means for setting the time needed for incubation are part of the assay system. Optionally, the assay medium is sterilized. Usually, the pH is adjusted to the required value. The method of the present invention may include mixing samples ($e.g.$ with other samples, but also with e.g. salts, buffering compounds, stabilizers, isotope-labeled compounds, fluorescence-labeled compounds and the like), concentrating and/or further diluting ($e.g.$ with diluting liquids such as water) samples prior to addition to the assay medium.

The term "CFU" is an abbreviation of Colony Forming Units and refers to the number of microorganisms, spores of microorganisms, partially germinated spores of microorganisms and/or vegetative cells capable of producing colonies of microorganisms. The concentration of said CFU's is expressed as Colony Forming Units per ml of assay medium ($CFU.ml^{-1}$) and is usually in the range of $1 \times 10^5$ to $1 \times 10^{12}$ $CFU.ml^{-1}$, preferably $1 \times 10^6$ to $1 \times 10^{10}$ $CFU.ml^{-1}$, more preferably $2 \times 10^6$ to $1 \times 10^9$ $CFU.ml^{-1}$, most preferably $5 \times 10^6$ to $1 \times 10^8$ $CFU.ml^{-1}$, or still more preferably $5 \times 10^6$ to $2 \times 10^7$ $CFU.ml^{-1}$.

The term "gelling agent" refers to a compound or substance that assists in changing a mixture into or taking on the form of a gel. Suitable examples of gelling agents are agar, alginic acid and salts thereof, carrageenan, gelatin, hydroxypropylguar and derivatives thereof, locust bean gum (Carob gum), processed eucheuma seaweed and the like.

In the context of the present invention, the term "indicator" refers to a substance used to measure (for example by change of color or fluorescence) the condition of an assay medium with respect to the presence of a particular component (for example an acid, a base, oxidizing or reducing agents). The indicator, upon changing from one state to another, provides a detectable signal such as a change in color or fluorescence. The indicator may be a pH-indicator, a redox-indicator or a combination thereof. The term indicator herein may also refer to two or more indicators. Examples of suitable indicators are well known to the skilled artisan (see handbook H.J. Conn's Biological Stains, R.D. Lillie ed., Baltimore, 1969). Particularly useful are indicators that, upon changing from one state to the other, provide a visually detectable signal such as a change in color or fluorescence. The amount of indicator in the assay medium is between 0.01 and 50 $g.l^{-1}$ assay medium, preferably between 0.1 and 10 $g.l^{-1}$, more preferably between 0.5 and 5 $g.l^{-1}$, most

preferably between 1 and 3 g.l$^{-1}$. Such indicators may be easily selected from handbooks such as 'H.J. Conn's Biological Stains', R.D. Lillie ed., Baltimore, 1969. Preferred indicators are pH-indicators and/or redox indicators. Examples of suitable indicators are Acid Blue 120, Acid Orange 51, Acid Yellow 38, Alizarin acid, Alizarin Blue, Azure A, Azure B, Basic Blue 3, Brilliant Black, Brilliant Cresyl Blue, Brilliant Crocein MOO, Brilliant Yellow, Bromocresol Green, Bromocresol Purple, Bromophenol Blue, Bromophenol Red, Bromothymol Blue, Chlorocresol Green, Congo Red, m-Cresol Purple, Gallocyanine, Indigo Carmine, Janus Green B, Litmus, Methylene Blue, Methyl Red, Nile Blue A, Nitrazol Yellow (also referred to as Nitrazine Yellow), o-Nitrophenol, p-Nitrophenol, 1-10 Phenanthroline, Phenolphthalein, Phenol Red, Safranine O, Thionin, Thymol Blue, Toluidine Blue and Xylenol Blue. Preferred indicators are indicators that change color in the pH range from 4.5 to 8.0, preferably from 5.0 to 7.5, more preferably from 5.5 to 7.0, preferably these are Bromocresol Green Bromocresol Purple, Bromothymol Blue, Methyl Red or Phenol Red, most preferably Bromocresol Purple or Bromothymol Blue.

The term "lactose-free sample" refers to a sample that is not milk or other lactose-comprising dairy products. Lactose-free sample refers to a sample in which the presence or absence of antibiotics needs to be determined and which is to be differentiated from samples of milk for which prior art antibiotic assays are widely applicable and recognized. Lactose-free also encompasses essentially lactose-free. Typically, a lactose-free sample contains less than 0.01% (w/w) of lactose, for example from 0.0001% (w/w) to 0.01% (w/w) of lactose, preferably from 0.00001% (w/w) to 0.005% (w/w) of lactose. Lactose-content may be determined according to methods known to the skilled person. Preferably lactose-content is determined according to ISO 22662:2007. Examples of lactose-free samples in the context of the present invention include samples from industrial waste streams from plants, including both aqueous and solid waste streams, samples from effluents and surface waters such as rivers, lakes, brooks and the like and samples from veterinary urine or manure. Lactose-free samples for use in the present invention can be from a fluid such as water, waste water, process water, sewage water, drinking water, water applied in *e.g.* swimming pools, saunas and greenhouses and cooling water. It can be water from agricultural sources, fisheries, ship ballast, cooling towers, waste water treatment plants, power plants, chemical industries such as textile industry, paper and pulp industry, printing industry, iron-steel industry, coke industry, petroleum industry, pesticide industry, paint industry, medical and dental industry, solvent industry, pharmaceutical industry, wood preserving chemicals industry, and food industry. The fluid can be an incoming effluent and/or stream, an outgoing effluent and/or stream, or an intermediate effluent and/or stream from any of the above sources.

The term "microorganism" refers to a microorganism that is sensitive towards the antibiotic, the presence or absence of which is to be determined by means of its growth.

The term "nutrient" as used herein refers to a nutritive substance or ingredient that promotes and/or is required for the growth of the microorganism. Suitable nutrients depend on the microorganism used in the assay medium. The assay medium may comprise two or more different nutrients. They include, but are not limited to, assimilable carbon sources such as carbohydrates such as *e.g.* glucose, fructose, sucrose, lactose and dextrose; assimilable nitrogen sources such as amino acids such as *e.g.* peptone or tryptone; sources of vitamins and growth factors such as beef or yeast extract; and sources of minerals such as earth alkaline metal salts such as salts of *e.g.* barium or calcium.

The terms "powdered milk" or "dried milk" refer to a manufactured dairy product made by evaporating milk to dryness. One purpose of drying milk is to preserve it; powdered milk has a far longer shelf life than liquid milk and does not need to be refrigerated, due to its low moisture content. Another purpose is to reduce its bulk for economy of transportation. Powdered milk and dairy products include such items as dry whole milk, nonfat (skimmed) dry milk, dry buttermilk, dry whey products and dry dairy blends.

In the context of the present invention, the term "spore" refers to a primitive usually unicellular often environmentally resistant dormant or reproductive body produced by microorganisms and capable of development into a new individual microorganism.

In a first aspect, the invention pertains to a method for determining the presence or absence of an antibiotic in a lactose-free sample that is essentially lactose-free and/or contains less than 0.01% (w/w) of lactose comprising the steps of:

(a) contacting said lactose-free sample with an assay medium which comprises a microorganism, a gelling agent and an indicator capable of detecting growth or inhibition of the microorganism to obtain an assembly of the lactose-free sample and the assay medium;
(b) incubating the assembly obtained in step (a) for a period of time sufficient to grow the microorganism in case no antibiotic is present in said lactose-free sample; and
(c) detecting growth or inhibition of growth of the microorganism with the indicator, characterized in that milk and/or powdered milk is added in step (a).

WO 2005/118837 A, which is considered to be the prior art closest to the subject-matter of the method of the invention, discloses a method for determining the presence or absence of an antibiotic in a sample comprising the steps of (a) contacting said sample with an assay medium which comprises a microorganism, a gelling agent and an indicator capable of detecting growth or inhibition of the microorganism to obtain an assembly of sample and assay medium; and (b) incubating the assembly obtained in step (a) for a period of time sufficient to grow the microorganism in case no antibiotic is present in said sample. See the abstract, the examples as well as claims 1, 6-8, 11.

Compared to said prior art disclosure, the subject-matter of the invention differs by the addition of milk and/or powdered milk. Surprisingly it was found that addition of milk or powdered milk to a lactose-free sample that is to be tested for the presence or absence of an antibiotic leads to improved accuracy and ease of detection as compared to performing the similar assay in the absence of (powdered) milk.

Carrying out microbiological assays for the determination of antibiotics in milk is, as described in the background of the invention, well known. Using microbiological assays for determining antibiotics in samples that are not stemming from the dairy industry has also been reported; usually these are aqueous lactose-free fluids. However there is no suggestion in the prior art to carry out, let alone improve, determination of antibiotics in lactose-free samples by adding (powdered) milk to said lactose-free samples. The mechanism behind this observation is not yet fully understood. Whether it is related to influences of the milk on the assay medium, the (growth of the) microorganism, a coloring effect on indicators or a chemical or physical interaction with the lactose-free sample to be analyzed is currently speculative and unsupported in the prior art.

In a first embodiment the microorganism is a strain of *Bacillus, Escherichia coli* or *Streptococcus.* In a further embodiment the microorganism is a thermophilic microorganism such as *Bacillus stearothermophilus* or *Streptococcus thermophilus.* The assay medium may comprise one or more types of microorganisms as detecting agents. The microorganism may be introduced in the assay medium as units capable of producing colonies, or CFUs. The growth of the microorganism in incubation step (b) is to take place during a predetermined period, preferably within a time span of 0.5 to 4 hours, more preferably between 1 to 3.5 hours, most preferably between 2.0 to 3.25 hours. Preferably the growth of the microorganism is conducted at a predetermined temperature, preferably the optimal growth temperature of the microorganism. When, for example, thermophilic microorganisms are used, said temperature preferably is between 40 and 70°C, more preferably between 50 and 65°C, most preferably between 60 and 64°C. Optionally, said reaction can be carried out with the aid of a thermostatic device. With the aid of the thermostatic device samples can be kept at a pre-set temperature, such as the temperature at which the microorganism shows sufficient growth. Preferably, said thermostatic device is designed in such a fashion that it can hold containers filled with assay medium. Optionally, the thermostatic device is coupled to a means for setting the time needed for incubation such that heating and/or cooling is stopped after lapse of a pre-set period. Alternatively, the time required for growth of the microorganism is equal to the time that is required for a calibration sample without any disinfectant to induce a change in the indicator.

In a second embodiment solids are removed from said lactose-free sample prior to step (a). Such removal may be effected using operations available to the skilled person, such as centrifugation, decantation, filtration and the like. Optionally, when said lactose-free sample contains many solids or even is a solid, the sample advantageously first is mixed with an aqueous solution prior to removal of solids. Said aqueous solution may be milk or powdered milk mixed with water.

In a third embodiment, the amount of lactose present in the combined lactose-free sample plus added milk or powdered milk is from 1 to 10% (w/w), preferably from 2 to 5% (w/w).

In a fourth embodiment a control test is carried out whereby a known amount of an antibiotic is added in step (a). Said antibiotic can be any antibiotic and preferably is penicillin G K.

In a fifth embodiment a control test is carried out whereby a known amount of a β-lactam degrading compound is added in step (a). Said β-lactam degrading compound can be any β-lactam degrading compound and preferably is a β-lactamase.

Although control tests are not mandatory for proper functioning of the method of the present invention, the skilled person understands that reliability of results improves when both the control tests of the fourth and fifth embodiments are carried out together with the method of the first aspect.

In a sixth embodiment the invention provides a method for determining the concentration of an antibiotic in a lactose-free sample comprising the steps of:

(a) preparing a dilution range of said lactose-free sample by the addition of milk;
(b) contacting each dilution obtained in step (a) with an assay medium comprising a microorganism, a nutrient and an indicator;
(c) incubating each mixture obtained in step (b) for a period of time to grow the microorganism in case no antibiotic is present in the sample;
(d) detecting growth or inhibition of growth of the microorganism with the indicator;
(e) determining the dilution with the highest dilution factor capable of inhibiting the growth of the microorganism;
(f) determining the concentration of the antibiotic in the dilution determined in step (e), and
(g) determining the concentration of the antibiotic in said lactose-free sample by multiplying the concentration determined in step (f) with the dilution factor determined in step (e) used to make the dilution.

Growth or inhibition of growth of the microorganism is detected by observing the presence or absence of a change of the indicator. Preferably, this is done for each dilution of the dilution range. When, for example the change of the indicator is a color change, the color change may be observed visually. However, in an embodiment of the invention the color change is determined using an arrangement that generates digital image data or an arrangement that generates analog image data and converts said analog image data into digital image data followed by interpretation of said digital image data by a computer processor. Such an arrangement, which may for instance be a sample-reading device such as a scanner coupled to a personal computer, is described in WO 03/033728. With such an arrangement it is possible to scan the bottom side of each of the samples in a test plate. The color and the brightness of the reflected light are registered in three variables, each describing one color component, for instance the so-called L*a*b* model. In the L*a*b* model, the color spectrum is divided in a two-dimensional matrix. The position of a color in this matrix is registered by means of the two variables "a" and "b". The variable L indicates the intensity (for instance, from light blue to dark-blue). It is possible to make a criterion comprising the a-value, b-value and L-value to make a composite function as follows:

$$Z = w_L.L + w_a.a + w_b.b$$

wherein $w_L$, $w_a$ and $w_b$ are weighting factors for the L-value, a-value and b-value, respectively. The values of these weighting factors can be calculated by means of "discriminent analysis", such that the group means show a maximum distance in relation to the spreading. By combining two or more of the color components in the L*a*b* model in a predetermined manner that depends on the type of disinfectant and the sample, an accurate detection is possible. In practice, a certain value of Z at which an assay should switch between positive and negative result is experimentally predetermined for each disinfectant. If desired, reading and computer equipment described above can be combined with heating equipment *e.g.* a thermostatic device as described in WO 2007/090683.

In a second aspect of the invention there is provided a kit for carrying out the method of the first aspect of the present invention, said kit comprising (a) a container with assay medium which comprises a microorganism, a gelling agent and an indicator capable of detecting growth or inhibition of the microorganism to obtain a mixture, and (b) a manual for carrying out the method of the first aspect of the invention such as a manual comprising instructions for determining the concentration of an antibiotic in a lactose-free sample that is essentially lactose-free and/or contains less than 0.01% (w/w) of lactose, and(c) a container comprising powdered milk.

EP 2 226 389 A1 discloses a kit comprising a test medium comprising a microorganism, a nutrient and an indicator (see paragraphs [0013]-[0017], and [0019]). GB 1 467 439 A discloses a test vessel containing bromescol purple, a spore culture and an agar medium (see example 1 as well as claims 34-38 and 41-42). WO 2005/118837 A1 discloses a kit suitable for the determination of the presence or absence of an antibiotic in a fluid comprising: (a) a container with a test medium comprising a test organism, at least one substance that provides a solid state, nutrients and an indicator.

(see the abstract as well as claim 11). However, none of these documents disclose a container comprising powdered milk.

Such a kit comprises one or more containers comprising assay medium as described above. The containers may be test tubes of any shape and size and from any material available, provided that observation of indicator changes is possible. Also, the containers may be wells such as those incorporated in micro-titer plates.

The manual for carrying out the method of the first aspect of the present invention should at least contain instructions for sample treatment including optional removal of solids by means of filtration or centrifugation and addition of milk or powdered milk as described in the first aspect of the invention.

In a further embodiment the kit comprises a sampling device that is a device with the aid of which fluid can be added to said assay medium. Preferably, such a device is a container, optionally with volume markings. More preferably, such a device is a syringe, a pipette or an automated pipetting system. Such a syringe or pipette may be designed in such a fashion that with only one mode of operation a predetermined volume can be withdrawn from the fluid to be analyzed. Optionally, systems known in the art with which more than one syringe or pipette can be operated with one single handling may be applied. It is the object of the second aspect of the present invention to provide a kit that allows for simple addition of the amounts of fluid to be added according to the first aspect of the invention.

In a further embodiment the kit comprises means for sealing said containers comprising assay medium during incubation.

In a further embodiment the kit comprises one or more containers with known amounts of an antibiotic to be used for carrying out a control test. Said antibiotic can be any antibiotic and preferably is penicillin G K.

In a further embodiment the kit comprises one or more containers with known amounts of a $\beta$-lactam degrading compound to be used for carrying out a control test. Said $\beta$-lactam degrading compound can be any $\beta$-lactam degrading compound and preferably is a $\beta$-lactamase.

In a further embodiment of the second aspect of the present invention, the kit comprises a thermostatic device, with the aid of which samples can be kept at a pre-set temperature, such as the temperature at which the microorganism shows sufficient growth. Preferably, said thermostatic device is designed in such a fashion that it can hold said containers filled with assay medium. Optionally the thermostatic device is coupled to a means for setting the time needed for incubation such that heating and/or cooling is stopped after lapse of a pre-set period.

In a further embodiment the kit comprises a data carrier loaded with a computer program suitable for instructing a computer to analyze digital data obtained from a sample-reading device. Said data carrier may be any carrier suitable for storing digital information such as a CD-ROM, a diskette, a DVD, a memory stick, a magnetic tape or the like. Advantageously, said data carrier loaded with a computer program provides for easy access to the latest available computer programs suitable for use in the method of the present invention.

In a third aspect of the invention there is provided a composition comprising a lactose-free sample containing less than 0.01% w/w lactose, a microorganism, a gelling agent, an indicator and one of milk and milk powder, wherein said lactose-free sample is selected from samples from industrial waste streams from plants, including both aqueous and solid waste streams, samples from effluents and surface waters and samples from veterinary urine or manure. Such composition is the result when the method of the first aspect of the invention is applied on a lactose-free sample, such as samples from industrial waste streams from plants, including both aqueous and solid waste streams, samples from effluents and surface waters such as rivers, lakes, brooks and the like and samples from veterinary urine or manure.

In a fourth aspect of the invention there is provided the use of Bromocresol Purple or Bromothymol Blue or a β-lactam degrading compound for determining the concentration of an antibiotic in a lactose-free sample that is essentially lactose-free and/or contains less than 0.01% (w/w) of lactose, wherein milk and/or powdered milk is added to said lactose-free sample. GB 1 467 439 A discloses the use of Bromocresol Purple for the determination of penicillin (page 2, left column, lines 58-63, and example 1). WO 2005/118837 A1 discloses the use of Bromocresol Purple or Bromothymol Blue (page 8, lines 5-6, and claim 9) discloses the use of Bromocresol Purple or Bromothymol Blue. US 5 591 599 A discloses the use of a β-lactamase (see page 2, left column, lines 15-19, and claim 7). None of these documents disclose the addition of milk and/or powdered milk.

**EXAMPLES**

**Example 1**

**Manual for determining antibiotics in waste water**

Following is a non-limiting example for what may be included as manual in a kit of the present invention.

Environment:

Make sure that the environment where the antibiotics determination will be executed is β-lactam free. If that cannot be guaranteed in the room itself a laminar flow cabinet can be used as alternative.

Equipment/accessories:

- Centrifuge for centrifuge tubes of 2 ml (rotation speed 15.000 rpm)
- Water bath which can be set on the temperatures of 37°C and 65°C
- pH meter
- Freezer with a separate compartment for storing the samples; a closed box in the freezer may be used as alternative
- Eppendorf pipet of 10-100 μl and 100-1000 μl
- Eppendorf pipet points of 100 μl and 1000 μl
- Centrifuge tubes of 2.0 ml
- Centrifuge tubes of 10 ml with screw cap
- Greiner centrifuge tubes of 50 ml with screw cap
- Milli Q water system
- Delvotest SP NT Ampoules (or comparable microbial antibiotic assays)
- Volumetric flasks (100 ml & 200 ml, sterilized and flushed 4 times with Millipore water)

Chemicals:

- 1N Sodium hydroxide (β-lactam free)
- 1N Hydrochloric acid (β-lactam free)
- β-lactamase BS (e.g. supplier AG scientific product number L-2467)
- Full fat milk (β-lactam free); as alternative powdered milk can be used, 1 gram powdered milk in 10 ml Milli Q water. The milk can be stored in the freezer (shelf life 3 months). Thaw the frozen milk slowly to room temperature before use. For all milk sources it is important to prove that the milk does not give a positive reaction in the assay.
- Penicillin G K

Solution preparation:

- Dilution water: Add 40 ml Milli Q water in a new Greiner centrifuge tube of 50 ml
- β-lactamase solution: weigh 50 mg in an centrifuge tube of 2 ml and add 2 ml Milli Q water. Adjust the amount if less or more is needed during the assay
- 1N Sodium hydroxide: weigh 1 gram NaOH in new Greiner centrifuge tube of 50 ml and dissolve in 25 ml Milli Q water and mix
- 1N Hydrochloric acid: Add 24 gram Milli Q water in a new Greiner centrifuge tube of 50 ml and add 0.9 ml concentrated HCl and mix
- 4 ppb penicillin G K stock solution: weigh exact 44 mg penicillin G K (90% pure) in a volumetric flask of 100 ml and dissolve and fill up with Milli Q water and mix (solution A). Add 1.0 ml of solution A in a volumetric flask of 100 ml and fill up with Milli Q water and mix (solution B). Add 1.0 ml of solution B in a volumetric flask of 100 ml and fill up with Milli Q water and mix (solution C). Weigh 5.0 gram of solution C in a Greiner centrifuge tube of 50 ml and add 45.0 gram milk and mix (4 ppb stock solution). Divide this solution over several centrifuge tubes of 10 ml, label every tube separate and freeze at -18°C (4 ml per tube and shelf life 3 months)
- 2 ppb penicillin G K test solution: Take a stock penicillin G K tube from the freezer and thaw slowly to room temperature. Mix 600 μl penicillin G K stock solution with 600 μl full milk and mix.

Sampling process:

During sampling, storage and transport it is crucial to assure that no β-lactam is introduced. To minimize the chance of contamination the following steps are needed:

1. Put on gloves and a clean lab coat to avoid contamination
2. Fill two Greiner centrifuge tubes of 50 ml with 40 ml sample and close the screw cap directly after adding
3. Label the sample tubes and make sure the text on the label is readable
4. Put the two tubes directly in a never used plastic bag and close it with a cable tie
5. Label also the plastic bag with the same information stated on the sample tubes itself
6. Store the two sample tubes in the plastic bag at -18°C, if the assay is not executed directly. Storage at -18 °C must be done within 15 minutes of sampling

Pretreatment:

If samples are frozen, defrost the sample by putting the sample one day before executing the analyses in the refrigerator (3-10°C).

1. Weigh 2 gram in a Greiner centrifuge tube of 50 ml and add 25 ml Milli Q water and mix until a homogeneous suspension; make certain that both phase are thoroughly mixed, especially in case of dry or dried samples
2. Shake the samples at least 10 minutes at room temperature
3. Adjust the pH to 6.2-6.8 with 1 N NaOH or 1 N HCl
4. Fill two 2 ml centrifuge tubes
5. Centrifuge 10 min at 14.000 rpm

The clear upper level will be used for further analyses.

Assay pre-handling:

There are three different sample pre-handling for the three different assays; depending on the requirements any or all of these assays may be performed:

1. Normal assay
2. Standard addition assay
3. Enzyme degradation assay

For the planning of the execution of these different pre-handling activities, take into account that the enzyme pre handling takes at least 2 hours lead time.

1. Normal assay pre-handling:

Add 600 $\mu$l clear sample solution in a centrifuge tube of 2.0 ml and add 600 $\mu$l milk and close directly the cap and mix; this mixture can directly be analyzed.

2. Standard addition assay pre handling:

Add 600 $\mu$l sample solution in a 2 ml centrifuge tube and add 600 $\mu$l 2 ppb penicillin G K test solution, close the cap and mix; this mixture can directly be analyzed.

3. Enzyme degradation assay pre-handling:

Add 700 $\mu$l clear sample solution in a centrifuge tube of 2.0 ml and add 100 $\mu$l B-lactamase solution and close directly the cap and mix. Place the centrifuge tube for 2 hours in a water bath of 37°C, cool down after two hours and add 700 $\mu$l milk and mix; this mixture can directly be analyzed.

Antibiotics determination execution:

Observe the general description as included with Delvotest SP NT Ampoules (or comparable). In the below Table an example is given of a hypothetical filled in sample table. In row one the blank (milk) and the 2 ppb penicillin G K test solution is added. The following codes for the different assays are applied in this example: N for normal assay; S for standard addition assay; E for enzyme degradation assay.

Table: Example of a sample table:

| Delvotest sample table | | | | | |
|---|---|---|---|---|---|
| A | B | C | D | E | row |
| blank | | 2 ppb | | Blank | 1 |
| N1 | N2 | | | | 2 |
| S1 | S2 | | | | 3 |
| E1 | E2 | | | | 4 |
| | | | | | 5 |

From all samples solutions 100 $\mu$l is brought in the Delvotest SP NT Ampoule with an Eppendorf pipet of 100 $\mu$l. With the point of the pipet the tube cover top of the ampoule will be penetrated and the 100 $\mu$l is added to the ampoule. Put the ampoules for 2.5 h in a water bath of 65°C. Seal the ampoule with tape. After 2.5 h the ampoules are removed from the water bath. Check if the blanks are yellow, if not prolong the time in the water bath with 30 min.

Interpretation of the results:

Yellow is negative and purple is positive. Positive means there is antimicrobial activity present in the sample which is higher than 2 ppb (calculated as penicillin G K).

*Normal assay results:*

- Purple means antimicrobial activity is > 2 ppb and the yellow < 2 ppb.

*Standard addition assay results:*

- If the sample is yellow in the normal assay and purple in the standard addition assay: The level of antimicrobial activity is < 2 ppb
- If the sample is yellow in the normal assay and yellow in the standard addition assay: There is a matrix effect results cannot be trusted. Dilution of the sample is needed.
- If the sample is purple in the normal assay the standard addition assay cannot be used.

*Enzyme degradation assay:*

- If the sample is purple in the normal assay and purple in the enzyme degradation assay. The results cannot be trusted. The sample needs to be reanalyzed with a higher amount of enzyme.
- If the sample is purple in the normal assay and yellow in the enzyme degradation assay: The level of antimicrobial activity is > 2 ppb.
- If the sample is yellow in the normal assay the enzyme degradation assay does not give any further information be used.

Calculation of the antimicrobial activity:

The results can be calculated as follows (limit of the method is 2 ppb, 2 ppb is 2 $\mu$g.l$^{-1}$):

- Sample: 2 g.25 ml$^{-1}$ which is 80 g.l$^{-1}$
- Diluted 2 times with milk => 40 g.l$^{-1}$
- If the sample colored purple means > 2 $\mu$g antimicrobial activity present in 40 gram.
- 2 $\mu$g.40 g$^{-1}$ => 50 $\mu$g.kg$^{-1}$=>>50 ppb antimicrobial activity.
- If yellow < 50 ppb antimicrobial activity.

**Example 2**

**Determining of antibiotic in a sample with and without added milk**

Two samples, a blank (water) and 2 ppb penicillin G K test solution were analysed using commercially available (DSM Food Specialties B.V., Delft, The Netherlands) Delvotest SP NT analogous to the supplier's manual. Two ampoules were filled with blank sample. To one milk (regular low-fat milk from a Dutch supermarket was used in the present example) was added according to the instruction as outlined in Example 1 and to the other the same amount of water was added. Also, two ampoules were filled with 2 ppb penicillin G K test solution and again to one milk was added according to the instruction as outlined in Example 1 and to the other the same amount of water was added. The ampoules filled with sample were incubated at 64°C +/- 2°C) and the colour of the agar in the ampoules was visually recorded at several points in time. The above experiment was performed in quintuple and the average observations were as summarized in the below Table.

| Time (min) | Blank | | Penicillin G K test solution | |
|---|---|---|---|---|
| | Added milk | Added water | Added milk | Added water |
| 60 | --- | --- | --- | --- |

(continued)

| Time (min) | Blank | | Penicillin G K test solution | |
|---|---|---|---|---|
| | Added milk | Added water | Added milk | Added water |
| 120 | --+ | --- | --- | --- |
| 150 | +++ | --- | --- | --- |
| 165 | +++ | --- | --- | --- |
| 180 | +++ | --+ | --- | --- |
| 195 | +++ | -++ | --- | --- |
| 210 | +++ | +++ | --- | --- |

As can be seen from the Table, blank samples without antibiotic present will lead to growing of the microorganism in the agar layer which in turn leads to a change in pH and a change in colour of the indicator from purple to yellow (symbols --+, - ++ and +++ in the Table denote partial to full change of colour from purple to yellow, respectively). Whereas samples of penicillin G K test solution do not show any colour change due to the inhibiting effect of the penicillin G K on growth of the microorganism in the blank sample the beginning of colour change can be observed already after 120 min in the presence of milk whereas this observation only can be made after 180 min in the absence of added milk. Furthermore, full colour change also takes place more rapidly in the presence of milk.

## Example 3

### Determining of antibiotic in a sample with and without added milk

Example 2 was repeated with various other samples such as in-process waste streams and extracts of fermentation mycelium filter cakes and in those cases where no antibiotic was present in the sample, the effect as observed in Example 2 (faster change of colour of the indicator in the agar layer of the Delvotest SP NT test ampoules in the presence of milk compared to the same in the absence of milk) was confirmed.

## Claims

1.  A method for determining the presence or absence of an antibiotic in a lactose-free sample that is essentially lactose-free and/or contains less than 0.01% (w/w) of lactose comprising the steps of:

    (a) contacting said lactose-free sample with an assay medium which comprises a microorganism, a gelling agent and an indicator capable of detecting growth or inhibition of the microorganism to obtain an assembly of the lactose-free sample and the assay medium;
    (b) incubating the assembly obtained in step (a) for a period of time sufficient to grow the microorganism in case no antibiotic is present in said lactose-free sample; and
    (c) detecting growth or inhibition of growth of the microorganism with the indicator,

    **characterized in that** milk and/or powdered milk is added in step (a).

2.  Method according to claim 1 wherein said assay medium further comprises nutrients.

3.  Method according to any one of claims 1 to 2 wherein said indicator is Bromocresol Purple or Bromothymol Blue.

4.  Method according to any one of claims 1 to 3 wherein said microorganism is thermophilic.

5.  Method according to claim 4 wherein said microorganism is *Bacillus stearothermophilus.*

6.  Method according to any one of claims 1 to 5 wherein said gelling agent is agar.

7.  Method according to any one of claims 1 to 6 wherein solids are removed from said lactose-free sample prior to step (a).

8. Method according to any one of claims 1 to 7 wherein the amount of lactose in said lactose-free sample plus said added milk or powdered milk is from 2 to 5% (w/w).

9. Method according to any one of claims 1 to 8 further comprising a control test selected from:

(i) a known amount of an antibiotic is added in step (a), or
(ii) a known amount of a β-lactam degrading compound is added in step (a), or
(iii) a combination of (i) and (ii).

10. A method for determining the concentration of an antibiotic in a lactose-free sample that is essentially lactose-free and/or contains less than 0.01% (w/w) of lactose comprising the steps of:

(a) preparing a dilution range of said lactose-free sample by the addition of milk;
(b) contacting each dilution obtained in step (a) with an assay medium comprising a microorganism, a nutrient and an indicator capable of detecting growth or inhibition of the microorganism to obtain a mixture;
(c) incubating each mixture obtained in step (b) for a period of time sufficient to grow the microorganism in case no antibiotic is present in the sample;
(d) detecting growth or inhibition of growth of the microorganism with the indicator;
(e) determining the dilution with the highest dilution factor capable of inhibiting the growth of the microorganism;
(f) determining the concentration of the antibiotic in the dilution determined in step (e), and
(g) determining the concentration of the antibiotic in said lactose-free sample by multiplying the concentration determined in step (f) with the dilution factor determined in step (e) used to make the dilution.

11. A kit comprising:

(a) a container with assay medium which comprises a microorganism, a gelling agent and an indicator capable of detecting growth or inhibition of the microorganism to obtain a mixture;
(b) a manual comprising instructions for determining the concentration of an antibiotic in a lactose-free sample that is essentially lactose-free and/or contains less than 0.01% (w/w) of lactose;
(c). a container comprising powdered milk.

12. Kit according to claim 11 wherein in step (b) the manual comprises instructions for carrying out the method of any one of claims 1 to 10.

13. A composition comprising a lactose-free sample containing less than 0.01% w/w lactose , a microorganism, a gelling agent, an indicator and one of milk and milkpowder, wherein said lactose free sample is selected from samples from industrial waste streams from plants, including both aqueous and solid waste streams, samples from effluents and surface waters and samples from veterinary urine or manure.

14. The use of Bromocresol Purple or Bromothymol Blue for determining the concentration of an antibiotic in a lactose-free sample that is essentially lactose-free and/or contains less than 0.01% (w/w) of lactose, wherein milk and/or powdered milk is added to said lactose-free sample.

15. The use of a β-lactam degrading compound for determining the concentration of an antibiotic in a lactose-free sample that is essentially lactose-free and/or contains less than 0.01% (w/w) of lactose, wherein milk and/or powdered milk is added to said lactose-free sample.

**Patentansprüche**

1. Verfahren zur Bestimmung des Vorliegens oder Fehlens eines Antibiotikums in einer lactosefreien Probe, die im Wesentlichen lactosefrei ist und/oder weniger als 0,01 Gew.-% Lactose enthält, umfassend die Schritte:

(a) Inkontaktbringen der lactosefreien Probe mit einem Assaymedium, das einen Mikroorganismus, einen Gelbildner und einen Indikator, mit dem sich Wachstum oder Hemmung des Mikroorganismus nachweisen lässt, umfasst, so dass eine Konstruktion der lactosefreien Probe und des Assaymediums erhalten wird;

(b) Inkubieren der in Schritt (a) erhaltenen Konstruktion über einen zum Kultivieren des Mikroorganismus hinreichenden Zeitraum, falls kein Antibiotikum in der lactosefreien Probe vorliegt; und
(c) Nachweisen von Wachstum oder Wachstumshemmung des Mikroorganismus mit dem Indikator,

**dadurch gekennzeichnet, dass** in Schritt (a) Milch und/oder Milchpulver zugegeben wird.

2. Verfahren nach Anspruch 1, wobei das Assaymedium ferner Nährstoffe umfasst.

3. Verfahren nach einem der Ansprüche 1 bis 2, wobei es sich bei dem Indikator um Bromkresolpurpur oder Bromthymolblau handelt.

4. Verfahren nach einem der Ansprüche 1 bis 3, wobei der Mikroorganismus thermophil ist.

5. Verfahren nach Anspruch 4, wobei es sich bei dem Mikroorganismus um *Bacillus stearothermophilus* handelt.

6. Verfahren nach einem der Ansprüche 1 bis 5, wobei es sich bei dem Gelbildner um Agar handelt.

7. Verfahren nach einem der Ansprüche 1 bis 6, wobei vor Schritt (a) Feststoffe aus der lactosefreien Probe entfernt werden.

8. Verfahren nach einem der Ansprüche 1 bis 7, wobei die Menge an Lactose in der lactosefreien Probe plus der zugegebenen Milch bzw. dem zugegebenen Milchpulver 2 bis 5 Gew.-% beträgt.

9. Verfahren nach einem der Ansprüche 1 bis 8, ferner umfassend einen Kontrolltest ausgewählt aus Folgendem:

(i) eine bekannte Menge eines Antibiotikums wird in Schritt (a) zugegeben, oder
(ii) eine bekannte Menge einer β-Lactam abbauenden Verbindung wird in Schritt (a) zugegeben, oder
(iii) eine Kombination von (i) (ii).

10. Verfahren zur Bestimmung der Konzentration eines Antibiotikums in einer lactosefreien Probe, die im Wesentlichen lactosefrei ist und/oder weniger als 0,01 Gew.-% Lactose enthält, umfassend die Schritte:

(a) Herstellen einer Verdünnungsreihe der lactosefreien Probe durch die Zugabe von Milch;
(b) Inkontaktbringen jeder in Schritt (a) erhaltenen Verdünnung mit einem Assaymedium, das einen Mikroorganismus, einen Nährstoff und einen Indikator, mit dem sich Wachstum oder Hemmung des Mikroorganismus nachweisen lässt, umfasst, so dass ein Gemisch erhalten wird;
(c) Inkubieren eines jeden in Schritt (b) erhaltenen Gemischs über einen zum Kultivieren des Mikroorganismus hinreichenden Zeitraum, falls kein Antibiotikum in der Probe vorliegt;
(d) Nachweisen von Wachstum oder Wachstumshemmung des Mikroorganismus mit dem Indikator;
(e) Bestimmen der Verdünnung mit dem höchsten Verdünnungsfaktor, durch die sich das Wachstum des Mikroorganismus hemmen lässt;
(f) Bestimmen der Konzentration des Antibiotikums in der in Schritt (e) bestimmten Verdünnung und
(g) Bestimmen der Konzentration des Antibiotikums in der lactosefreien Probe durch Multiplizieren der in Schritt (f) bestimmten Konzentration mit dem in Schritt (e) bestimmten zum Herstellen der Verdünnung verwendeten Verdünnungsfaktor.

11. Kit, umfassend:

(a) einen Behälter mit Assaymedium, das einen Mikroorganismus, einen Gelbildner und einen Indikator, mit dem sich Wachstum oder Hemmung des Mikroorganismus nachweisen lässt, umfasst, so dass ein Gemisch erhalten wird;
(b) ein Handbuch, das Anweisungen zur Bestimmung der Konzentration eines Antibiotikums in einer lactosefreien Probe, die im Wesentlichen lactosefrei ist und/oder weniger als 0,01 Gew.-% Lactose enthält, umfasst;
(c) einen Behälter, der Milchpulver umfasst.

12. Kit nach Anspruch 11, wobei in Schritt (b) das Handbuch Anweisungen zur Durchführung des Verfahrens gemäß einem der Ansprüche 1 bis 10 umfasst.

**EP 3 368 681 B1**

13. Zusammensetzung, umfassend eine lactosefreie Probe, die weniger als 0,01 Gew.-% Lactose enthält, einen Mikroorganismus, einen Gelbildner, einen Indikator und entweder Milch oder Milchpulver, wobei die lactosefreie Probe ausgewählt ist aus Proben von Industrieabfallströmen von Pflanzen, einschließlich sowohl wässriger als auch fester Abfallströme, Proben von Abwässern und Oberflächenwassern und Proben von veterinärem Urin oder Dung.

14. Verwendung von Bromkresolpurpur oder Bromthymolblau zur Bestimmung der Konzentration eines Antibiotikums in einer lactosefreien Probe, die im Wesentlichen lactosefrei ist und/oder weniger als 0,01 Gew.-% Lactose enthält, wobei Milch und/oder Milchpulver zur lactosefreien Probe gegeben wird.

15. Verwendung einer β-Lactam abbauenden Verbindung zur Bestimmung der Konzentration eines Antibiotikums in einer lactosefreien Probe, die im Wesentlichen lactosefrei ist und/oder weniger als 0,01 Gew.-% Lactose enthält, wobei Milch und/oder Milchpulver zur lactosefreien Probe gegeben wird.


**Revendications**

1. Méthode destinée à déterminer la présence ou l'absence d'un antibiotique dans un échantillon exempt de lactose qui est essentiellement exempt de lactose et/ou contient moins de 0,01% (p/p) de lactose, comprenant les étapes de:

   (a) mettre en contact ledit échantillon exempt de lactose avec un milieu de dosage qui comprend un microorganisme, un agent gélifiant et un indicateur capable de détecter la croissance ou l'inhibition du microorganisme, afin d'obtenir un assemblage de l'échantillon exempt de lactose et du milieu de dosage;
   (b) incuber l'assemblage obtenu dans l'étape (a) pendant une période de temps suffisante pour cultiver le microorganisme dans le cas où aucun antibiotique n'est présent dans ledit échantillon exempt de lactose ; et
   (c) détecter la croissance ou l'inhibition de la croissance du microorganisme à l'aide de l'indicateur ; **caractérisée en ce que** du lait et/ou du lait en poudre est ajouté dans l'étape (a).

2. Méthode selon la revendication 1, dans laquelle ledit milieu de dosage comprend en outre des nutriments.

3. Méthode selon l'une quelconque des revendications 1 à 2, dans laquelle ledit indicateur est le pourpre de bromocrésol ou le bleu de bromothymol.

4. Méthode selon l'une quelconque des revendications 1 à 3, dans laquelle ledit microorganisme est thermophile.

5. Méthode selon la revendication 4, dans laquelle ledit microorganisme est *Bacillus stearothermophilus.*

6. Méthode selon l'une quelconque des revendications 1 à 5, dans laquelle ledit agent gélifiant est l'agar-agar.

7. Méthode selon l'une quelconque des revendications 1 à 6, dans laquelle les matières solides sont éliminées à partir dudit échantillon exempt de lactose préalablement à l'étape (a).

8. Méthode selon l'une quelconque des revendications 1 à 7, dans laquelle la quantité de lactose dans ledit échantillon exempt de lactose plus ledit lait ou lait en poudre ajouté va de 2 à 5% (p/p).

9. Méthode selon l'une quelconque des revendications 1 à 8, comprenant en outre un test de contrôle choisi parmi :

   (i) une quantité connue d'un antibiotique est ajoutée dans l'étape (a), ou
   (ii) une quantité connue d'un composé de dégradation de β-lactame est ajoutée dans l'étape (a), ou
   (iii) une combinaison de (i) et (ii).

10. Méthode destinée à déterminer la concentration d'un antibiotique dans un échantillon exempt de lactose qui est essentiellement exempt de lactose et/ou contient moins de 0,01% (p/p) de lactose, comprenant les étapes consistant à :

    (a) préparer une gamme de dilution dudit échantillon exempt de lactose par l'addition de lait ;
    (b) mettre en contact chaque dilution obtenue dans l'étape (a) avec un milieu de dosage qui comprend un microorganisme, un nutriment et un indicateur capable de détecter la croissance ou l'inhibition du microorganisme, afin d'obtenir un mélange ;

(c) incuber chaque mélange obtenu dans l'étape (b) pendant une période de temps suffisante pour cultiver le microorganisme dans le cas où aucun antibiotique n'est présent dans l'échantillon ;

(d) détecter la croissance ou l'inhibition de la croissance du microorganisme à l'aide de l'indicateur ;

(e) déterminer la dilution ayant le facteur de dilution le plus grand capable d'inhiber la croissance du microorganisme ;

(f) déterminer la concentration en antibiotique dans la dilution déterminée dans l'étape (e) ; et

(g) déterminer la concentration en antibiotique dans ledit échantillon exempt de lactose en multipliant la concentration déterminée dans l'étape (f) par le facteur de dilution déterminé dans l'étape (e) utilisé pour préparer la dilution.

11. Kit, comprenant :

(a) un conteneur ayant un milieu de dosage qui comprend un microorganisme, un agent gélifiant et un indicateur capable de détecter la croissance ou l'inhibition du microorganisme, afin d'obtenir un mélange ;

(b) un manuel comprenant des instructions destinées à déterminer la concentration d'un antibiotique dans un échantillon exempt de lactose qui est essentiellement exempt de lactose et/ou contient moins de 0,01% (p/p) de lactose ;

(c) un conteneur comprenant du lait en poudre.

12. Kit selon la revendication 11, dans lequel, dans l'étape (b), le manuel comprend des instructions destinées à mettre en oeuvre la méthode selon l'une quelconque des revendications 1 à 10.

13. Composition comprenant un échantillon exempt de lactose contenant moins de 0,01% p/p de lactose, un microorganisme, un agent gélifiant, un indicateur, et l'un parmi du lait et de la poudre de lait, où ledit échantillon exempt de lactose est choisi parmi des échantillons issus de courants de déchets industriels issus d'usines, y compris des courants de déchets aqueux ainsi que solides, des échantillons issus d'effluents et d'eaux de surface et des échantillons issus de fumier ou d'urine vétérinaire.

14. Utilisation de pourpre de bromocrésol ou de bleu de bromothymol afin de déterminer la concentration d'un antibiotique dans un échantillon exempt de lactose qui est essentiellement exempt de lactose et/ou contient moins de 0,01% (p/p) de lactose, où du lait et/ou du lait en poudre est ajouté audit échantillon exempt de lactose.

15. Utilisation d'un composé de dégradation de β-lactame afin de déterminer la concentration d'un antibiotique dans un échantillon exempt de lactose qui est essentiellement exempt de lactose et/ou contient moins de 0,01% (p/p) de lactose, où du lait et/ou du lait en poudre est ajouté audit échantillon exempt de lactose.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 2056581
- DE 3613794
- EP 0005891 A
- EP 0285792 A
- EP 2226389 A
- GB 1467439 A
- WO 9418343 A
- WO 2005118837 A
- WO 03033728 A
- WO 2007090683 A
- EP 2226389 A1
- WO 2005118837 A1
- US 5591599 A

**Non-patent literature cited in the description**

- **PETTAS ; KARAYANNIS.** *Anal. Chim. Acta,* 2004, vol. 522, 275-280
- handbook H.J. Conn's Biological Stains. 1969
- H.J. Conn's Biological Stains. 1969